# NEW EUROPEAN PATENT SPECIFICATION

(11) **EP 1 536 783 B2**
(45) Date of publication and mention of the opposition decision: **24.08.2011**
(45) Mention of the grant of the patent: 06.08.2008
(21) Application number: 03772489.5
(22) Date of filing: 10.09.2003
(51) Int. Cl.: A61K 31/404, A61K 9/16

(54) **SOLID FORMULATIONS COMPRISING AN INDOLINONE COMPOUND**
FESTE FORMULIERUNGEN ENTHALTEND EINE INDOLINON VERBINDUNG
FORMULATIONS SOLIDES CONTENANT UN COMPOSE INDOLINONE

(30) Priority: 10.09.2002 US 421133 P
(43) Date of publication of application: 08.06.2005
(73) Proprietor: Pfizer Italia S.r.l., 04010 Latina (IT)
(72) Inventor: GATTI, Paolo, 20014 Genova (IT)
(74) Representative: Ruddock, Keith Stephen
(86) International application number: PCT/IB2003/005293
(87) International publication number: WO 2004/024127

(56) References cited:
- WO-A-01/37820
- WO-A2-03/035009
- ABDULLAH E C ET AL: "The use of bulk density measurements as flowability indicators" POWDER TECHNOLOGY 03 MAY 1999 SWITZERLAND, vol. 102, no. 2, 3 May 1999 (1999-05-03), pages 151-165, XP002272427 ISSN: 0032-5910

## Description

### FIELD OF THE INVENTION

The instant invention provides a composition for a pyrrole substituted 2-indolinone. Also described are methods of making and using the formulation of the invention.

### BACKGROUND OF THE INVENTION

The following description of the background of the invention is provided to aid in understanding the invention, but is not admitted to describe or constitute prior art to the invention.

Various methods are available for administering therapeutic agents to a patient. Such methods include parenteral, oral, ocular, nasal, topical, and transmucosal administration.
WO 01/37820 relates to formulations for pharmaceutical agents ionizable as free acids or free bases. Abdullah et al. [Powder Technology 102 (1999) 151-165] report on the use of bulk density measurements as flowability indicators.

There is a need in the art for a stable, uniform formulation of indolinones which can be readily formed into dosage forms and which is substantially free of the disadvantages of formulations disclosed in the prior art. An object of the invention is to provide a stable indolinone formulation which can be readily formed into an oral capsule or tablet.

### SUMMARY OF THE INVENTION

The invention relates to a granular composition as claimed in claim 1.

The granular composition comprises the compound: 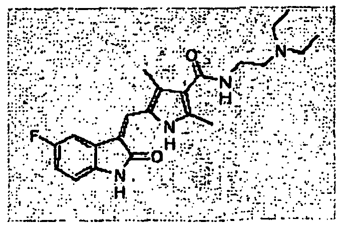 in the form of its L-malate salt.

### DETAILED DESCRIPTION OF THE PREFERRED EMBODIMENTS

The instant invention features a granular composition comprising an indolinone. In particular, the composition aids the administration of the indolinone to patients in need of treatment.

The granular composition of claim 1 comprises the indolinone of the formula:

| | |
|---|---|
| 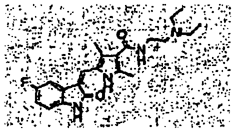 | 5-(5-fluoro-2-oxo-1,2-dihydroindol-3-ylidenemethyl)-2,4-dimethyl-1H-pyrrole-3-carboxylic acid(2-diethylaminoethyl)amide: in the form of its L-malate salt |

In the invention composition a therapeutically effective amount of an indolinone is utilized.

As used herein, a "pharmaceutically acceptable carrier" refers to a carrier or diluent that does not abrogate the biological activity and/or properties of the administered compound while facilitating administration by, for example, stabilizing or solubilizing the compound.

The term "pharmaceutically acceptable" or "pharmaceutical" as used herein refers to solutions or components of the formulation that do not prevent the therapeutic compound from exerting a therapeutic effect and do not cause unacceptable adverse side effects. Examples of pharmaceutically acceptable reagents are provided in The United States Pharmacopeia The National Formulary, United States Pharmacopeial Convention, Inc., Rockville, MD 1990 and FDA Inactive Ingredient Guide 1990, 1996 issued by the Division of Drug Information Resources (both are hereby incorporated by reference herein, including any drawings). Unacceptable side effects vary for different diseases. Generally, the more severe the disease the more toxic effects which will be tolerated. Unacceptable side effects for different diseases are known in the art.

The composition of the invention is suitable for oral administration, the composition is solid

The term "solubilized" as used herein refers to dissolving of a substance in a fluid and/or adsorption of fluid molecules on the surface of the substance to assist in such dissolving: In one aspect, "solubilized" refers to hydration of a substance in water.

The term "molar equivalent" as used herein refers to equal or similar molar amounts of a test substance as compared to a reference substance.

The term "acid solution" as used herein refers to an acidic solution, typically one which has a pH lower than 7 and is capable of reacting with a basic solution. Preferably the acid in the acid solution is selected from the group consisting of hydrochloric acid, sulfuric acid, formic acid, lactic acid, malic acid, maleic acid, succinic acid, acetic acid, methane sulfonic acid, benzene sulfonic acid, phosphoric acid, malonic acid and the like, and suitable combinations of two or more hereof.

The term "buffer" as used herein refers to a substance, preferably in a solution, that resists a change of quality. Preferably a buffer is a solution that resists a change to a pH, such as a substance in a solution capable of neutralizing both acids and bases and therefore maintaining an original acidity or basicity of a solution. Suitable buffers include acetate, citrate, phosphate buffer, ascorbate, hydrochloric acid buffer, Tris-HCl buffer, sodium phosphate, sodium carbonate, sodium hydroxide, glutamate, glycine, Tris base buffers, and the like, and suitable combinations of two or more hereof. Most preferably, the buffer is sodium phosphate buffer.

In one embodiment, the buffer pH is three pH units lower than the pK_{b} of the ionizable substituted indolinone. Preferably, the buffer has a molarity (i.e., molar concentration, measured in moles per liter (M)) between 0.01 M and 0.1 M.

The term "pK_{b}" as used herein refers to the negative logarithm of the basicity constant, the basicity constant being the product of the concentration of the hydroxyl ion and the concentration of the conjugated acid, divided by the concentration of the base (the basicity constant is also sometimes referred to as the equilibrium constant).

In an additional aspect, the invention provides pharmaceutically acceptable compositions containing an indolinone. In preferred embodiments, the invention formulation suitable for oral administration is encapsulated in a hard gelatin capsule.

The capsule sizes that may be used in the practice of the preferred embodiments of the present invention are capsules that range from size 00 to size 4.

In a further preferred embodiment of the method of preparing a formulation, the method also includes sterilizing the formulation. Preferably, the sterilizing is done by gamma irradiation, autoclaving or aseptic processing.

Generally, bulk density is the weight of a unit volume of material. Bulk density is also known as "apparent density." Bulk density is typically expressed as a weight:volume ratio, for example, kilograms per liter (Kg/L) or grams per cubic centimeter (g/cm²). Bulk desntiy of a material can be measured by techniques that are well known in the art, by measuring the weight and volume of the material. See the Examples section of this application for exemplary techniques for measurement of bulk density.

"Tap density" is also used to assess the properties of the composition of the invention. Tap density can be measured by techniques that are well known in the art, and apparati for measuring tap density are commercially available. Briefly, the material is subjected to a series of "taps" that cause the material to be compacted, or reduced in volume. The density (weight/volume) of the "tapped" material is the tap density. Typically, the tapping is carried out until the tap density measurement has stabilized. For example, as shown in Comparative Example section of this application, material was tapped 500 times, volume was measured (volume measurement #1). The material was then tapped an additional number of times so that total number of taps was 1250, and then a second volume measurement (#2) was taken. If measurement #1 and #2 differed by greater than 2 milliliters, then the material was tapped an additional 1250 times.

The ratio of tap density to bulk density of the composition is from about 1.10 to about 1.30. In still other preferred embodiments, the ratio of tap density to bulk density is from about 1.10 to about 1.30. In other preferred embodiments, the ratio of tap density to bulk density is from about 1.10 to about 1.33. In other preferred embodiments, the ratio of tap density to bulk density is from about 1.10 to about 1.25. In other preferred embodiments, the ratio of tap density to bulk density is from about 1.10 to about 1.20. Preferably, the ratio of tap density to bulk density is from about 1.10 to about 1.15.

Preferably the composition is prepared such that no more than 55% of the particles have a size less than 250 microns. More preferably the mean particle size of the particles in the formulation is in the range of about 106-250 microns; or wherein the mean particle size of the particles in the formulation is in the range of about 150-250 microns or in the range of about 250-350 microns, or no more than about 55% of the particles of the formulation have a size between about 106 and about 250 microns, or no more than about 50% or about 45% or about 40% or about 35% or about 30% of the particles of the formulation have a size between about 106 and about 250 microns.

The bulk density of the composition is from about 2 to about 8 fold greater than the bulk density of the indolinone itself. Preferably the bulk density of the composition is about 2 to about 8 fold greater than the bulk density of the malate salt of the indolinone itself, or the bulk density of the formulation is about 3 to about 8 fold, or about 4 to about 8 fold, or about 5 to about 8 fold, or about 6 to about 8 fold greater than the bulk density of the indolinone itself.

Further preferably the bulk density of the composition is at least about 2 fold greater than the bulk density of the indolinone itself or the bulk density of the composition is at least about 3 fold, or at least about 4 fold greater than the bulk density of the indolinone itself, or the bulk density of the composition is at least about 5 fold greater than the bulk density of the indolinone itself, or the bulk density of the composition is at least about 6 fold or at least about 7 fold greater than the bulk density of the indolinone itself.

### Methods of Treatment

The composition is effective in treating or preventing an abnormal condition in a patient in need of such treatment. The patient is preferably a mammal and more preferably a human.

The term "preventing" as used herein refers to administering the formulation to a patient before the abnormal condition manifests itself in that patient.

The term "treating" as used herein refers to the method of the invention having a therapeutic effect and at least partially alleviating or abrogating the abnormal condition in the organism (e.g., patient).

The term "therapeutic effect" as used herein refers to inhibition of the abnormal condition. The term "therapeutic effect" also refers to the inhibition of factors causing or contributing to the abnormal condition. A therapeutic effect relieves to some extent one or more of the symptoms of the abnormal condition.

The term "mammal" as used herein preferably refers to the organisms of the class known as "mammalia", such as mice, rats, rabbits, guinea pigs, goats, sheep, horses, cows, dogs, cats, monkeys, apes, humans, and the like; more preferably dogs, cats, monkeys, apes, humans, and the like; and most preferably humans.

The term "abnormal condition" refers to a function in the cells or tissues of a patient that deviates from normal functions in that patient. An abnormal condition can relate to cell proliferation (e.g., be a cell proliferative disorder) as described herein.

The term "cell proliferative disorder" as used herein refers to a disorder where an excess cell proliferation of one or more subset of cells in a multicellular organism occurs resulting in harm (e.g., discomfort or decreased life expectancy) to the multicellular organism. The excess cell proliferation can be determined by reference to the general population and/or by reference to a particular patient (e.g., at an earlier point in the patient's life). Hyper-proliferative cell disorders can occur in different types of animals and in humans, and produce different physical manifestations depending upon the affected cells. Hyper-proliferative cell disorders include without limitation cancers, blood vessel proliferative disorders, fibrotic disorders, autoimmune disorders, and the like. Cell proliferative disorders suitable for treatment in accordance with the present invention include without limitation cancers (e.g., erythroblastoma, glioblastoma, meningioma, astrocytoma, melanoma, myoblastoma, breast cancers, gastric cancers, ovarian cancers, renal cancers, hepatic cancers, pancreatic cancers, bladder cancers, thyroid cancers, prostate cancers, colorectal cancers, solid tumor cancers, colon cancer, brain cancer, blood cancers, bone cancers, liver cancer, kidney cancer, stomach cancer, lung cancer, Kaposi's sarcoma, non-small cell lung cancer, skin cancer, and the like, non-small cell lung cancers, and the like).

In reference to the treatment of abnormal conditions caused, in whole or in part, by a cell proliferative disorder, a therapeutic effect refers to one or more of the following: (a) reducing tumor size; (b) inhibiting (e.g, slowing or stopping) tumor metastasis; (c) inhibiting tumor growth; and (d) relieving to some extent one or more of the symptoms associated with the abnormal condition.

Thus, the composition of the present invention is effective in methods of preventing or treating an abnormal condition in a patient in need of treatment comprising orally administering the composition to said patient.

In preferred embodiments of a method of preventing or treating an abnormal condition in a patient in need of treatment with an oral composition.

In yet other preferred embodiments of a method of preventing or treating an abnormal condition in a patient in need of treatment with an oral composition, the patient is a mammal, preferably a human.

Further, the composition of the present invention is effective in methods of preventing or treating an abnormal condition in a patient in need of treatment comprising orally administering to the patient the composition.

### DOSAGE

The composition of the present invention includes an active ingredient in an amount sufficient to achieve the intended purpose; i.e., the modulation of protein kinase (PK) activity or the treatment or prevention of a PK-related disorder.

The above referenced protein kinase related disorder is selected from the group consisting of an EGFR related disorder, a PDGFR related disorder, an IGFR related disorder and a flk related disorder.

The above referenced protein kinase related disorder is a cancer selected from the group consisting of squamous cell carcinoma, sarcomas such as Kaposi's sarcoma, astrocytoma, glioblastoma, lung cancer, bladder cancer, colorectal cancer, gastrointestinal cancer, head and neck cancer, melanoma, ovarian cancer, prostate cancer, breast cancer, small-cell lung cancer, leukemia and glioma in a further aspect of this invention.

The above referenced protein kinase related disorder is selected from the group consisting of diabetes, a hyper- proliferation disorder, von Hippel-Lindau disease, restenosis, fibrosis, psoriasis, osteoarthritis, rheumatoid arthritis, an inflammatory disorder, mastocytosis and angiogenesis in yet another aspect of this invention.

Additional disorders which may be treated or prevented using the compounds of this invention are immunological disorders such as autoimmune disease (AIDS) and cardiovasular disorders such as atherosclerosis.

More specifically, a therapeutically effective amount means an amount of compound effective to prevent, alleviate or ameliorate symptoms of disease or prolong the survival of the subject being treated.

Determination of a therapeutically effective amount is well within the capability of those skilled in the art, especially in light of the detailed disclosure provided herein.

For the compound used in the methods of the invention, the therapeutically effective amount or dose can be estimated initially from cell culture assays. Then, the dosage can be formulated for use in animal models so as to achieve a circulating concentration range that includes the IC₅₀ as determined in cell culture (i.e., the concentration of the test compound which achieves a half-maximal inhibition of the PK activity). Such information can then be used to more accurately determine useful doses in humans.

Toxicity and therapeutic efficacy of the compound described herein can be determined by standard pharmaceutical procedures in cell cultures or experimental animals, e.g., by determining the IC₅₀ and the LD₅₀ (both of which are discussed elsewhere herein) for a subject compound. The data obtained from these cell culture assays and animal studies can be used in formulating a range of dosage for use in humans. The dosage may vary depending upon the dosage form employed and the route of administration utilized. The exact dosage can be chosen by the individual physician in view of the patient's condition. (See e.g., Fingl, et al., 1975, in "The Pharmacological Basis of Therapeutics", Ch. 1 p.1).

Therapeutic compounds should be more potent in inhibiting receptor tyrosine kinase activity than in exerting a cytotoxic effect. A measure of the effectiveness and cell toxicity of a compound can be obtained by determining the therapeutic index; i.e., IC₅₀/LD₅₀. IC₅₀, the dose required to achieve 50% inhibition, can be measured using standard techniques such as those described herein. LD₅₀, the dosage which results in 50% toxicity, can also be measured by standard techniques as well(Mossman, 1983, J. Immunol. Methods, 65:55-63), by measuring the amount of LDH released (Korzeniewski and Callewaert, 1983, J. Immunol. Methods, 64:313; Decker and Lohmann-Matthes; 1988, J. Immunol. Methods, 115:61), or by measuring the lethal dose in animal models. Compounds with a large therapeutic index are preferred. Thus, the preferred dosage of the compound, contemplated for use in the invention requires the therapeutic index to be greater than 2, preferably at least 10, more preferably at least 50.

Dosage amount and interval may be adjusted individually to provide plasma levels of the active species, which are sufficient to maintain the kinase modulating effects. These plasma levels are referred to as minimal effective concentrations (MECs). The MEC can be estimated from in vitro data; e.g., the concentration necessary to achieve 50-90% inhibition of a kinase may be ascertained using the assays described herein. Dosages necessary to achieve the MEC will depend on individual characteristics and route of administration. HPLC assays or bioassays can be used to determine plasma concentrations.

Dosage intervals can also be determined using MEC value. The compound should be administered using a regimen that maintains plasma levels above the MEC for 10-90% of the time, preferably between 30-90% and most preferably between 50-90%.

In cases of local administration or selective uptake, the effective local concentration of the drug may not be related to plasma concentration and other procedures known in the art may be employed to determine the correct dosage amount and interval.

The amount of a composition administered will, of course, be dependent on the subject being treated, the severity of the affliction, the manner of administration, the judgment of the prescribing physician, etc.

In general, a "therapeutically effective amount" refers to that amount of an agent or its metabolite which is effective to prevent, alleviate, reduce or ameliorate symptoms of disease and/or the undesired side effects attributable to treatment of disease with another agent or its metabolite, or to prolong the survival of the patient being treated. More particularly, in reference to the treatment of cancer, a therapeutically effective amount refers to that amount which has the effect of (1) reducing the size of (or preferably eliminating) the tumor; (2) inhibiting (that is, slowing to some extent, preferably stopping) tumor metastasis; (3) inhibiting to some extent (that is slowing to some extent, preferably stopping) tumor growth; and/or, (4) relieving to some extent (or preferably eliminating) one or more symptoms associated with the cancer and/or one or more undesired side effects attributable to treatment of the cancer with another agent or its metabolite.

In addition to the above general definition, by a "therapeutically effective amount" of an agent is meant any amount administered in any manner and in any treatment regime as may be currently recognized in the medical arts or as may come about as the result of future developments regarding the use of these agents.

A "treatment regime" refers to specific quantities of the ionizable substituted indolinone contemplated for use in this invention) administered at set times in a set manner over an established time period.

When referring to "set times" of administration within a treatment regime, "consecutive days" means consecutive calendar days; i.e., Monday, Tuesday, Wednesday, etc. "Staggered" days means calendar days with other calendar days between them, e.g., without limitation, Monday, Wednesday, Saturday, etc.

Furthermore, with regard to a "therapeutically effective amount" of the indolinone, the phrase refers to an amount of the compound sufficient to inhibit the growth, size and vascularization; i.e., angiogenesis and/or vasculogenesis, of tumors during the "recovery" periods, i.e., the periods in a treatment regime when no other chemotherapeutic agent is being administered to a patient.

In a presently preferred embodiment, the indolinone composition dose is administered during rest periods when no other agent is being administered to a patient. Tablets

Methods of making tablets are known in the art. The three basic methods for the preparation of compressed tablets or capsules are the wet granulation method, the dry granulation method and direct compression (tablets). (Ansel et al., "Pharmaceutical Dosage Forms and Drug Delivery Systems" 1995, Williams and Wilkins, which is incorporated by reference in its entirety).

Wet granulation is a widely employed method for the production of compressed tablets or capsules. The steps required in the the preparation of tablets or capsules by this method may be separated as follows: (1) weighing and blending the ingredients (2) preparing the wet granulation (3) screening the damp mass into pellets or granules (4) drying (5) dry screening (6) lubrication and blending and (7) tableting by compression or encapsulating. In weighing and blending the active ingredient and any filler, disintegrating agent required in the formulation are weighed and mixed thoroughly. The total amount of the disintegrant is not always added to the drug-diluent mixture, but a portion is reserved for later addition with the lubricant to the prepared granulation of the drug. Granulation is accomplished by adding a liquid binder or an adhesive to the powder mixture, passing the wetted mass through a screen of the desired mesh size, drying the granulation and then passing through a second screen of smaller mesh size to reduce further the size of the granules. Generally the wet granulation is pressed through a mesh screen. After all of the material has been converted into granules, the granulation is spread evenly on large pieces of paper and dried. After drying, a dry lubricant is generally added to the granulation so that each granule is covered with lubricant. The composition is then pressed into tablets or encapsulated.

In the dry granulation method, the granulation is formed not by moistening or adding a binding agent to the powdered drug mixture byt by compressing large masses of the mixture and subsequently crushing and sizing these pieces into smaller granules. By this method either the active ingredient or the diluent should have cohesive properties in order for the large masses to be formed. After weighing and mixing the ingredients the powder is "slugged" or compressed into large flat tablets or pellets. The slugs are broken up by hand or by a mill and passed through a screen of desired mesh size for sizing. Lubricant is added and tablets are prepared by compression or the drug mixture is encapsulated.

### Packaging

The compositions may, if desired, be presented in a pack or dispenser device, such as an FDA approved kit, which may contain one or more unit dosage forms containing the active ingredient. The pack may for example comprise metal or plastic foil, such as a blister pack. The pack or dispenser device may be accompanied by instructions for administration. The pack or dispenser may also be accompanied by a notice associated with the container in a form prescribed by a governmental agency regulating the manufacture, use or sale of pharmaceuticals, which notice is reflective of approval by the agency of the form of the compositions or of human or veterinary administration. Such notice, for example, may be of the labeling approved by the U.S. Food and Drug Administration for prescription drugs or of an approved product insert. Compositions may also be prepared, placed in an appropriate container, and labeled for treatment of an indicated condition. Suitable conditions indicated on the label may include treatment of a tumor, inhibition of angiogenesis, treatment of fibrosis, diabetes, and the like.

### SYNTHESIS EXAMPLES

The compound used in this invention, as well as the precursor 2-oxindoles and aldehydes, may be readily synthesized using techniques well known in the chemical arts. The syntheses of the compound utilised in the present invention is disclosed in U.S. Serial No. 10/076,140, filed February 15, 2002, PCT Application No. PCT/US02/04407, and published PCT application WO O1/60814; and U.S. Serial No. 10/281,985, filed Aug. 13, 2002, claiming priority to U.S. Serial No. 60/312,353, filed August 15, 2001; Yet, it will be appreciated by those skilled in the art that other synthetic pathways for forming the compounds of the invention are available and that the following is offered by way of example and not limitation.

### FORMULATION EXAMPLES

Generally, the composition of the present invention is prepared by combining the active pharmaceutical ingredient (API) with a pharmaceutically acceptable diluent, binder, disintegrant and lubricant.

### Method for Making a Granular Composition

1. Mix all ingredients, except magnesium stearate and 50% croscarmellose sodium in a high shear granulator.
2. Granulate using purified water.as the granulating fluid.
3. Dry granules in a fluid bed granulator.
4. Mill dried granules with an oscillating sieve to appropriate granule size.
5. Blend the sieved granule with the remaining croscarmellose sodium (50%) in an appropriate size.
6. Add magnesium stearate and blend.

### Example 1 (reference)

| Composition of 5-(5-fluoro-2-oxo-21,2-dihydro-indol-3-ylidenemethyl)-2,4-dimethyl-1H-pyrrolo-3-carboxylic acid (2-diethylamino-ethyl)-amide hard gelatin capsules | | | | |
|---|---|---|---|---|
| Ingredient Name | Concentration in Granulation (%w/w) | Amount in 50 mg Capsule (mg) | Amount in 75 mg Capsule (mg) | Amount in 200 mg Capsule (mg) |
| API | 65.0 | 50.0 | 75.0 | 200.0 |
| Mannitol | 23.5 | 18.1 | 27.2 | 72.4 |
| Croscaramellose Sodium^{e} | 6.0 | 4.6 | 6.9 | 18.4 |
| Povidone (K-25) | 5.0 | 3.8 | 5.7 | 15.2 |
| Magnesium Stearate | 0.5 | 0.38 | 0.57 | 1.52 |
| Capsule | - | Size 1 | Size 3 | Size 0 |

In Example 1, the free base form of the compound was used. The bulk density of the granular composition of the formulation used to make a 50 mg capsule was 0.44 kg/L and the tap density was 0.60 kg/L. The bulk density of the granular composition of the formulation used to make a 75 mg capsule was 0.46 kg/L and the tap density was 0.63 kg/L. The ratio of tap to bulk density for both formulations was 1.36 kg/L.

### Example 2 (reference)

| Composition of 5-(5-fluoro-2-oxo-1,2-dihydro-indol-3-ylidenemethyl)-2,4-dimethyl-1H-pyrrole-3-carboxylic acid (2-diethylamino-ethyl)-amide L-malate hard gelatin capsules | | |
|---|---|---|
| Ingredient Name/Grade | Concentration in Granulation (% w/w) | Amount in 50 mg Capsule (mg) |
| API | 75.0 | 66.800^{e} |
| Mannitol | 13.5 | 12.024 |
| Croscaramellose Sodium | 6.0 | 5.344 |
| Povidone (K-25) | 5.0 | 4.453 |
| Magnesium Stearate | 0.5 | 1.445 |
| Capsule | - | Size 3 |

### Example 3

| Composition of 5-(5-fluoro-2-oxo-1,2-dihydro-indol-3-ylidenemethyl)-2,4-dimethyl-1H-pyrrole-3-carboxylic acid (2-diethylamino-ethyl)-amide L-malate hard gelatin capsules | | | | |
|---|---|---|---|---|
| Ingredient Name/Grade | Concentration in Granulation (% w/w) | Amount in 25 mg Capsule (mg) | Amount in 50 mg Capsule (mg) | Amount in 100 mg Capsule (mg) |
| API^{a} | 40.0 | 33.400^{d} | 66.800^{c} | 133.6^{b} |
| Mannitol | 47.5 | 39.663 | 79.326 | 158.652 |
| Croscaramellose Sodium^{e} | 6.0 | 5.010 | 10.020 | 20.04 |
| Povidone (K-25) | 5.0 | 4.175 | 8.350 | 16.700 |
| Magnesium Stearate | 1.5 | 1.252 | 2.504 | 5.008 |
| Capsule | - | Size 3 | Size 1 | Size 0 |

| | | | | |
|---|---|---|---|---|
| ^{a} Drug substance quantity required for the batch will be ajusted to have 100% of labeled strength for capsules. Appropriate adjustment will be made to mannitol quantity to keep the same fill weight for each strength. ^{b} Quantity equivalent to 100 mg free base. ^{c} Quantity equivalent to 50 mg free base. ^{d} Quantity equivalent to 25 mg free base. ^{e} Half intraganular half extragranular. | | | | |

The bulk density of the L-malate salt 5-(S-fluoro-2-oxo-1,2-dihydro-indol-3-ylidenemethyl)-2,4-dimethyl-1H-pyrrole-3-carboxylic acid (2-diethylamino-ethyl)-amide, by itself, was measured to be between about 0.11 +/- 0.1. The bulk density of a formulation batch (different than the batches discussed below in the Comparative Example) was measured to be about 0.68 kg/L for the 50 mg capsule, and the tap density was about 0.81 kg/L. For the 25 mg capsules, the bulk density was about 0.64 kg/L and the tap density was about 0.8 kg/L. Therefore, the ratio of the bulk density of the formulation to the density of the L-malate salt is about 0.68/0.11 = 6.81 for the 50 mg capsules, and 0.64/0.11 = 5.81 for the 25 mg capsules.

**Example 4**

| **Composition of SU011248 L-Malate Salt Drug Product: 12.5 mg Hard Gelatin Capsules** | | |
|---|---|---|
| **Ingredient Name/Grade** | **12.5-mg Capsule** | |
| | **Concentration in Granulation (% w/w)** | **Amount in 12.5-mg Capsule (mg)** |
| Formulation Code | J-010398-AC | J-010398-AC-00 |
| SU011248 L-malate salt^{a} | 15.2 | 16.70^{b} |
| Mannitol NF | 72.7 | 80.00 |
| Croscarmellose sodium NF | 6.0 | 6.60 |
| Povidone (K-25) USP | 5.1 | 5.60 |
| Magnesium stearate NF | 1.0 | 1.10 |
| Total Fill Weight | 100 | 110.0 |
| Capsule | - | Swedish Orange, Size 4 |

| | | |
|---|---|---|
| ^{a} Drug substance quantity required for the batch will be adjusted to contain 100% of labeled strength. Appropriate adjustment will be made to mannitol quantity to keep the same fill weight for each strength. ^{b} Quantity equivalent to 12.5 mg free base | | |

The bulk density of this formulation was about 0.67 kg/L and the tap density was 0.77 kg/L. The size distribution of the particles are as follows:

| Particle Size (µm) | Percentage |
|---|---|
| >1000 | 0.26 |
| 1000-710 | 6.50 |
| 710-500 | 5.1 |
| 500-250 | 19.0 |
| 250-106 | 54.0 |
| <106 | 15.2 |

### Example 5 (Reference): Formulation for 5-[(Z)-(5-fluoro-2-oxo-1,2-dihydro-3H-indol-3-ylidene)methyl]-N-[2-hydroxy-3-morpholin-4-ylpropyl]-2,4-dimethyl-1H-pyrrole-3-carboxamide

5-[(Z)-(S-fluoro-2-oxo-1,2-dihydro-3H-indol-3-ylidene)methyl]-N-[2-hydroxy-3-morpholin-4-ylpropyl]-2,4-dimethyl-1H-pyrrole-3-carboxamide is formulated as as the maleate salt. The compound is formulated in the same fashion as described above in the section entitled "Method for Making a Granular Composition" and in Examples 1 - 4. The compound is formulated as either the (R) isomer, the (S) isomer or as mixtures of both isomers.

The maleate salt of this compound was determined to have a bulk density of about 0.05-0.07 Kg/l.

### Comparative Example

A capsule containing a formulation comprising 75% w/w of the malate salt of 5-(5-fluoro-2-oxo-1,2-dihydro-indol-3-ylidenemethyl)-2,4-dimethyl-1H-pyrrole-3-carboxylic acid (2-diethylamino-ethyl)-amide was developed. *See* Table 1, below. During the capsule production using this amount of the API, however, excessive sticking problems were observed during the capsule filling process. The sticking problems occurred in the hopper, filling heads and other moving parts of the capsule filling machine. The sticking problems necessiatated halting the capsule filling process several times to clean machine parts.

An improved formulation was developed as shown in Table 1, below. The new formulation comprises 40% w/w 5-(5-fluoro-2-oxo-1,2-dihydro-indol-3-ylidenemethyl)-2,4-dimethyl-1H-pyrrole-3-carboxylic acid (2-diethylamino-ethyl)-amide L-malate and 1.5 % w/w magensium stearate. The improved formulation did not exhibit the sticking problems observed with the 75% w/w formulation.

**Table 1: Comparison of the 75% w/w and the 40% w/w formulation***

| **Ingredient Name/Grade** | **concentration (% w/w)** | | **Ingredient Name/Grade** | **concentration (% w/w)** |
|---|---|---|---|---|
| **API** | 75.00 | | **API** | 40.00 |
| **Mannitol** | 13.50 | | **Mannitol** | 47.50 |
| **Sodium croscarmelose (in)** | 3.00 | | **Sodium croscarmelose (in)** | 3.00 |
| **Povidone K25 (in)** | 5.00 | | **Povidone K25 (in)** | 5.00 |
| **Sodium croscarmelose (out)** | 3.00 | | **Sodium croscarmelose (out)** | 3.00 |
| **Magnesium Stearate (out)** | 0.50 | | **Magnesium Stearate (out)** | 1.50 |

| | | | | |
|---|---|---|---|---|
| The reduction of API was compensated for by an increase in mannitol amount | | | | |

### Granulation and Blending Procedures

Three batches were produced: two using 150 g API and one using 200 g API.

For wet granulation, a high shear granulator (Key international KG 5) equipped with a 3 L bowl was used. The 200 g API batch (500 g dry mixture) filled about 45% of the bowl volume.

The API and the intragranular excipients (addition order: mannitol, povidone and croscaramellose sodium) were mixed into the high shear granulator for about 2 minutes using impeller speed of about 300 rpm and chopper speed of about 4,000 rpm. The residual water content (L.O.D.) of the dry mixture was measured on a representative sample and expressed as percent loss of mass upon drying of the sample (test conducted using a thermobalance with drying temperature 110° C, until sample constant weight is reached).

Water was added through a funnel to the mixture; impeller speed was about 400 rpm and chopper speed was about 5000-6000 rpm. Water was added and material was kneaded until the granules were wet but not sticky. The ordinary skilled artisan would be able to ascertain the point at which the granules are wet but not sticky.

The amounts of water added and granulation times are summarized in the following table.

**Table 2: Details of the Granulation Process**

| **API Amount (g)** | **Dry mixture L.O.D.** | **Water amount** | **Water % (on whole formulation)** | **Granulation time** |
|---|---|---|---|---|
| 150 | 1.65% | **90 overwetted** | 24.0 | 6'30" |
| 150 | 1.57% | 70 | 18.7 | 8' |
| 200 | 1.41% | 92.2 | 18.4 | 8' |

The drying process was conducted on a Uni-Glatt fluid bed dryer with inlet air temperature at 60°C, until an outlet air temperature of 40°C was reached. The L.O.D. evaluation was done and the drying process stopped if a value equal to or less than that of the starting dry mixture was obtained.

The drying processes for the second and third batches were conducted with flap at 25-30% and lasted 19 and 22 minutes, respectively. The L.O.D. values at the end of the process were below the required limit.

The drying process was stopped when the L.O.D. was below 2.5%.

Dry granules were sized through mill (fluidair granulmill junior) equipped with 1 mm screen (round holes); the process was conducted with mill speed of 1500 rpm. At the end of the process, the L.O.D. was recorded and the values were inside the limits proposed.

For each granulation, the bulk and tap density and particle size distribution were recorded; for particle size distribution determination, Sonic Sieve Sifting equipment was used. *See* below for representative values for tap density and particle size distribution.

The granules from the batches that were not overwetted were combined and a final blend of 746.3 g was obtained. L.O.D. and density measurements and particle size distribution test were performed on the final blend. *See* below for data.

### Capsule Filling Procedures

### Manual

Using the above final blend, capsules of 25, 50 and 100 mg (calculated based on free base) were prepared.

The capsules were filled by hand using a volumetric filling head from a Zanasi AZ5 machine. Before starting encapsulation, twenty dosing weights were recorded to evaluate correct set up of the filling head.

During the filling, the dosing weight was periodically checked to guarantee as much uniformity as possible. Capsules and tooling were of size 3 for 25 mg (calculated based on free base), of size 1 for 50 mg and of size 0 for 100 mg.

### Automatic

An automatic filling machine was equipped with size 3 dies on the feeding system and with size one holder on the capsule disc, to prepare 50 mg capsules. The operating speed was about 3000 capsules/hour; set up of the four tamping systems was at 20 mm (lowest pressure).

### Results and Discussion

### Granulation

The following tables report the densities and particle size distribution values for the two batches used to prepare the final blend.

**Table 3: Density values for granulations**

| **Batch** | **2204-007** | **2204-014** | **Final mixture** |
|---|---|---|---|
| **Bulk density (g/mL)** | 0.61 | 0.62 | 0.63 |
| **Tapped density (g/m L)** | 0.73 | 0.75 | 0.77 |

**Table 4: Particle size distribution values for granulations**

| **Batch** | **2204-007** | **2204-014** | **Final mixture** |
|---|---|---|---|
| **Mesh** | **% retained** | **% retained** | **% retained** |
| **20** | 0.47 | 0.19 | 0.38 |
| **40** | 17.57 | 11.59 | 11.30 |
| **60** | 33.62 | 20.23 | 23.17 |
| **80** | 20.51 | 21.75 | 19.94 |
| **100** | 7.31 | 12.92 | 10.83 |
| **200** | 13.49 | 24.69 | 19.28 |
| **fines** | 7.22 | 8.64 | 10.64 |

The two granulation batches showed good densities and flowed very well.

### Tap, Bulk Density and Particle Size Distribution Determinations

The tap and bulk density determinations are performed as follows:
(a) A 250 mL glass cylinder is filled with formulation granules to the 100 - 200 mL volume mark.
(b) The mass of the cyclinder is recorded and the bulk density is determined by calculating the ratio of the mass of the formulation granules to the volume of the formulation granules.
(c) The cylinder is then put into a tapping apparatus and the volume is and recorded after 10; 500 and 1250 taps.
(d) The ratio between the mass of the formulation granules and its volume after 1250 taps is the tap density.
(e) If the difference of volume after 500 and 1250 taps is higher than 2 mL, another1250 taps are applied before reading the volume once again and calculating the tap density.

The particle size distributions are determined by using sieves (1000, 710, 500, 250, 106 micrometer) and a sieving apparatus which vibrates for a specified period of time (e.g., 3 minutes).

### Capsule filling

The final mixture obtained was used to fill manually 25 capsules of 25 mg (calculated based on free base) using a filling head from Zanasi AZ5 capsule filling machine, equipped with size 3 doser. The theoretical filling weight was 83.3 mg; the average weight of empty size 3 shells was 48.7 mg and the filled capsules average weight was 131.0 mg.

First, 550 capsules of 25 mg (granules batch 2204-014) were filled. For these capsules, the average weight of empty shells was 48.7 mg; the average weight of filled capsules was 130.1 mg. Eighty capsules of 50 mg (fill weight 166.6 mg, granules batch 2204-014) were prepared using size 1 shells having an average weight of 74.4 mg; the average weight of filled capsules was 241.2 mg. Fifty capsules of 100 mg (fill weight 333.2 mg, granules batch 2204-014) were prepared using size 0 shells having an average weight of 95.4 mg; the average weight of filled capsules was 428.0 mg.

The results of the automatic capsule filling (granules from batch 2204-014) test were favorable, even if the dies used (the smallest available) overshot the target filling weight.

It was possible to obtain a minimum filling weight of about 181.5 mg (theoretical 166.6 mg for 50 mg dose) and the uniformity of weight obtained was excellent (average weight relative standard deviation (CV) <1.0%) indicating very good flowability of the mixture. In other preferred embodiments, the CV may be from about 1 to about 6%; from about 6 - 4%; preferably from about 2 to about 4%; more preferably from about 1 to about 3%; most preferably < 1%. About 3500 capsules were produced.

## Claims

1. A granular composition consisting of
• 40% w/w of 5-(5-fluoro-2-oxo-1,2-dihydroindol-3-ylidenemethyl)-2,4-dimethyl-1H-pyrrole-3-carboxylic acid (2-diethylamino-ethyl)-amide L-malate;
• 47.5% w/w of mannitol;
• 6.0% w/w of croscarmellose sodium;
• 5.0% w/w of povidone (K-25); and
• 1.5% w/w of magnesium stearate:
wherein half the amount of croscarmellose sodium is intragranular, and half the amount of croscarmellose sodium together with the whole amount of magnesium stearate are extragranular.

## Patentansprüche

1. Granulierte Zusammensetzung, bestehend aus
• 40% G/G 5-(5-Fluor-2-oxo-1,2-dihydroindol-3-ylidenmethyl)-2,4-dimethyl-1H-pyrrol-3-carbonsäure-(2-diethylamino-ethyl)-amid-L-malat
• 47,5% G/G Mannitol,
• 6% G/G Croscarmellose-Natrium,
• 5% G/G Povidon (K-25)
• und 1,5% G/G Magnesiumstearat;
wobei die Hälfte der Menge von Croscarmellose-Natrium intragranular und die Hälfte der Menge von Croscarmellose-Natrium zusammen mit der gesamten Menge Magnesiumstearat extragranular ist.

## Revendications

1. Composition granulaire consistant en
• 40 % en poids/poids de L-malate de (2-diéthyl-amino-éthyl)-amide d'acide 5-(5-fluoro-2-oxo-1,2-dihydro-indole-3-ylidèneméthyl)-2,4-diméthyl-1H-pyrrole-3-carboxylique ;
• 47,5 % en poids/poids de mannitol ;
• 6,0 % en poids/poids de croscarmellose sodique ;
• 5,0 % en poids/poids de povidone (K-25) ; et
• 1,5 % en poids/poids de stéarate de magnésium ;
dans laquelle la moitié de la quantité de croscarmellose sodique est intragranulaire, et la moitié de la quantité de croscarmellose sodique conjointement avec la quantité totale de stéarate de magnésium est extragranulaire.
